# EUROPEAN PATENT APPLICATION

(11) **EP 4 840 458 A1**
(43) Date of publication of application: **18.11.2026**
(21) Application number: 26166891.7
(22) Date of filing: 23.03.2026
(51) Int. Cl.: A61K 36/899, A23L 33/105, A61K 8/9789, A61P 17/02, A61Q 19/00

(54) **EXTRACTION AND PURIFICATION PROCESS OF A BIOLOGICALLY ACTIVE FRACTION DERIVED FROM COMMON WHEAT**

(30) Priority: 15.05.2025 IT 202500011010
(71) Applicant: Farmaceutici Damor S.P.A.., 80145 Napoli (IT)
(72) Inventor: ROMANO, Eugenia, 80132 Naples (IT); MAGLIONE, Barbara, 20900 Monza (IT)
(74) Representative: Lunati & Mazzoni S.r.L.

(57) **Abstract**

An extraction and purification process of a biologically active fraction derived from common wheat having a molecular weight lower than 30 kDa is provided, comprising, in order, at least the steps of germinating wheat seeds on water or on a natural or artificial substrate until sprouts are obtained; heat treating the sprouts thereby obtaining treated sprouts; macerating the treated sprouts in an aqueous solution thereby obtaining a solid residue; separating the solid residue from the aqueous solution until a clear solution is obtained; sterilizing the clear solution by removing portions having a molecular weight greater than 30 kDa; wherein, during sterilization, portions having a molecular weight between 3 and 10 kDa are also removed so as to obtain a biologically active fraction consisting exclusively of portions having a molecular weight lower than 3 kDa and between 10 and 30 kDa.

## Description

The present invention relates to a process for the extraction and purification of a biologically active fraction derived from common wheat of the type specified in the preamble of the first claim.

In particular, the present invention relates to a process for the extraction and purification of a biologically active fraction named Rigenase^{®} and derived from Triticum vulgare or aestivum, also known as common wheat.

The use of saccharidic fractions of plant origin, in particular polysaccharides and oligosaccharides, for their antioxidant and regenerative activities has been widely studied due to their beneficial biological properties. These plant-derived compounds are recognized for their ability to modulate essential biological processes, such as cell proliferation, extracellular matrix (ECM) synthesis, cell migration, and the reduction of oxidative stress, all of which are fundamental processes in tissue repair and regeneration.

Plant-derived polysaccharides, such as those isolated from *Aloe vera, Glycyrrhiza glabra (licorice), Centella asiatica and Triticum vulgare,* have been shown to promote tissue regeneration by stimulating collagen synthesis and the production of other extracellular matrix components. This results in the formation of new tissue and accelerated wound healing. Polysaccharides are particularly effective in modulating inflammation and enhancing the wound healing process, promoting fibroblast proliferation and keratinocyte migration, two key mechanisms involved in skin repair and tissue regeneration.

Furthermore, plant saccharidic fractions possess significant antioxidant properties, which help counteract oxidative damage, one of the critical factors in tissue regeneration. By reducing oxidative stress, such compounds promote cellular protection and facilitate the regeneration of damaged tissues.

Consequently, saccharidic fractions of plant origin have found application in a variety of therapeutic treatments and medical devices, such as creams, gels, and wound dressings, used to promote the healing of lesions, pressure ulcers, burns, and other skin injuries.

Therefore, the incorporation of plant saccharidic fractions into formulations intended for tissue regeneration has a well-established traditional use and represents an effective strategy to improve therapeutic outcomes and reduce recovery times in patients, making such compounds valuable both in the clinical and pharmaceutical fields.

**In** particular, *Triticum vulgare* is a cereal widely cultivated worldwide and is known not only for its nutritional value, but also for the presence of phytochemicals having therapeutic and cosmetic potential.

**In** recent years, various extraction techniques have been optimized in order to isolate different bioactive fractions from this plant matrix for specific clinical applications. Such processes are described, for example, in documents WO2016043684A1 and WO2007096448A1.

A particular example of a process, described in patent document WO2013182551A1, makes it possible to obtain a saccharidic fraction from germinated wheat, having a specific molecular weight between 3.000 and 30.000 daltons, and may be summarized in the following essential steps.

The process starts from the germination of wheat seeds, which preferably takes place in the dark, with high humidity and at low temperatures, until seedlings of a few centimeters are obtained.

The germinated plant material is then subjected to a heat treatment at high temperatures (between 85 and 150°C) for a defined period, optionally with the addition of water, in order to prepare it for extraction.

This is followed by maceration of the treated material in water, preferably under slightly acidic conditions (pH 2-7) and at low temperature (2-10°C). This stage extracts the components from the germinated wheat.

The liquid is then separated from the solid by methods such as filtration or pressing, thereby obtaining a clear "crude extract". This extract may be sterilized. It is also possible to reuse the liquid extract to macerate new seedlings, in a process referred to as "curling", in order to increase the yield.

The crucial step is purification based on molecular weight. Fractions having a molecular weight lower than 3.000 daltons and those higher than 30.000 daltons are removed from the crude extract. This is typically carried out by ultrafiltration, using membranes with specific cut-offs (first 3 kDa to discard the low molecular weight fraction, then 30 kDa to discard the high molecular weight fraction). The desired active fraction is the one that elutes in the second stage and has a molecular weight between 3.000 and 30.000 daltons.

Finally, the solution containing the purified active fraction may be left in aqueous solution or isolated by drying or lyophilization.

In summary, the process focuses on germinating the wheat, subjecting it to heat treatment, extracting the components in acidified and cold water, separating the liquid and, fundamentally, purifying it by molecular weight in order to isolate only the saccharidic fraction in the 3-30 kDa range, which is absent in non-germinated seeds and is considered the biologically active part.

The known technique described includes some important drawbacks.

In particular, the described processes do not make it possible to obtain extracts having effective antioxidant and moisturizing activities. Therefore, such extracts are poorly suited for use in non-pharmaceutical compositions (i.e. medical devices) and in cosmetic compositions, including antioxidant, moisturizing and humectant activities.

Indeed, for example, the extract obtained with the process described in WO2013182551A1 mainly exhibits its effectiveness in the context of stimulating cell proliferation and in mechanisms related to tissue healing.

Document WO2016043684A1 focuses on the use of an aqueous extract of Triticum vulgare for the topical treatment of hemorrhoids, the main properties of which are anti-inflammatory, decongestant and hemostatic, contributing to the rapid reduction of pain, inflammation and bleeding in the anal area. Finally, document WO2007096448A1 instead proposes a nutraceutical interpretation of wheat extract, making it possible to obtain a natural coffee substitute derived from roasted and ground *Triticum aestivum,* from which a caffeine-free beverage rich in nutrients is obtained.

In this situation, the technical problem underlying the present invention is to devise a process for the extraction and purification of a biologically active fraction derived from common wheat capable of substantially overcoming at least part of the aforementioned drawbacks.

Within the scope of this technical problem, an important aim of the invention is to obtain a process for the extraction and purification of a biologically active fraction derived from common wheat that makes it possible to obtain an extract having high antioxidant, moisturizing and humectant capacities.

Another important aim of the invention is to provide a process for the extraction and purification of a biologically active fraction derived from common wheat that makes it possible to obtain a fraction particularly suitable for use in non-pharmaceutical compositions, for example in medical devices, and in cosmetic compositions, including antioxidant, moisturizing and humectant activities.

The technical problem and the specified aims are achieved by a process for the extraction and purification of a biologically active fraction derived from common wheat as claimed in the appended claim 1.

Preferred technical solutions are highlighted in the dependent claims.

The features and advantages of the invention are clarified below by the detailed description of preferred embodiments of the invention, with reference to the accompanying drawings, in which:
**Fig. 1** shows the linear correlation of the antioxidant content with respect to the Oxygen Radical Antioxidant Capacity (ORAC) value of the fraction, wherein the correlation analysis was performed using the GraphPad Prism 6.01 software (La Jolla, CA 92037, USA);
**Fig. 2** illustrates the inhibition of AAPH-induced erythrocyte hemolysis by the fraction, wherein the erythrocyte suspension was incubated with AAPH at 37 °C in the absence (indicated in the graph by dashed line) and in the presence of the fraction (indicated in the graph as a continuous line) or of ascorbic acid (indicated in the graph by dotted line), and wherein each value, from which spontaneous hemolysis has been subtracted, represents the mean ± SD of three separate experiments;
**Fig. 3** is a comparative representation of the samples analysed to evaluate the in vitro dose-related protection against dehydration on human keratinocytes, and wherein it is highlighted that the highest effect is observed at the concentration of 5 mg/ml (74.33% protection) of the fraction.

In the present document, measurements, values, shapes and geometric references (such as perpendicularity and parallelism), when associated with words such as "about" or other similar terms such as "approximately" or "substantially", are to be understood as allowing for measurement errors or inaccuracies due to production and/or manufacturing errors and, above all, as allowing for a slight deviation from the value, measurement, shape or geometric reference with which they are associated. For instance, these terms, if associated with a value, preferably indicate a divergence of not more than 10% of the value.

Furthermore, when used, terms such as "first", "second", "upper", "lower", "main" and "secondary" do not necessarily identify an order, a priority of relationship or relative position, but may simply be used to more clearly distinguish between different components.

Unless otherwise specified, as reflected in the following discussions, terms such as "processing", "computing", "determination", "computation", or the like are considered to refer to the action and/or processes of a computer or similar electronic computing device that manipulates and/or transforms data represented as physical, such as electronic quantities of records of a computer system and/or memories, into other data similarly represented as physical quantities within computer systems, records, or other information storage, transmission, or display devices.

The measurements and data reported in this text are to be considered, unless otherwise indicated, as performed in the International Standard Atmosphere ICAO (ISO 2533:1975).

The present document describes a method for obtaining a specific fraction derived from Triticum vulgare (common wheat) through a cold aqueous extraction process. This extract proves to be particularly effective in promoting tissue regeneration and wound healing when applied topically in conventional formulations due to its antioxidant, moisturizing and emollient activities.

Indeed, its main mechanism of action is to form a protective barrier over wounds against the external environment, thereby creating favorable conditions for rapid and proper skin re-epithelialization.

More specifically, the fraction obtained with the process according to the invention is a specific aqueous extract of *Triticum vulgare* consisting predominantly of a class of homologous oligosaccharides having different degrees of glucose polymerization and a molecular weight between 10 and 30 kDa and lower than 3 kDa. From a quantitative standpoint, the percentage by weight as reducing sugars (glucose) is approximately 60% of the total.

The extracted fraction may be applied in various forms, including aqueous solutions, suspensions or emulsions, to the affected area. The active fraction is particularly effective in conventional non-pharmacological and cosmetic compositions for the treatment of skin wounds, ulcers, burns and other conditions requiring enhanced tissue regeneration and/or protection, wherein it is preferably used in concentrations between 5% and 10% by weight of the final composition.

The present invention relates to a new fraction derived from wheat germ, specifically obtained through a particular aqueous maceration process. This fraction, having a molecular weight lower than 3 kDa, exhibits a range of unexpected biological properties, including emollient and antioxidant activities. Unlike traditional growth factors, this fraction is of plant origin and has the chemical structure of a polysaccharide rather than a protein. These distinctive characteristics make the fraction particularly valuable for the development of cosmetic products and medical devices, especially for the treatment of wounds, ulcers and other skin conditions.

The process therefore comprises a plurality of stages. Preferably, the stages are carried out in the order as described below.

In particular, the process comprises at least one germination stage.

In the germination stage, common wheat seeds are germinated on water or on a natural or artificial substrate until sprouts are obtained. Germination preferably takes place in the absence of light, on lignocellulosic substrates such as poplar sawdust, at temperatures between 10 and 20 °C and with relative humidity above 60%, until the sprouts reach a height between 4 and 7 cm.

The process then comprises a heat treatment stage.

In the heat treatment stage, the obtained sprouts are subjected to heat treatment. Preferably, the heat treatment is carried out in an autoclave at 121 °C for 60 minutes. The process further comprises a maceration stage.

In the maceration stage, the treated sprouts are macerated in an aqueous solution, thereby obtaining a solid residue.

The maceration is preferably carried out in an aqueous solution having a pH between 2 and 4, at temperatures between 2 and 10 °C, for a period ranging between 24 and 72 hours.

The process therefore also comprises a separation stage.

In the separation stage, the solid residue is separated from the aqueous solution, thereby obtaining a clear solution.

Preferably, the separation takes place by a coarse filtration stage by pressing or mechanical filtration.

The process therefore comprises a sterilization or purification stage.

**In** the sterilization stage, the solution is preferably sterilized by removing at least portions having a molecular weight greater than 30 kDa.

Moreover, advantageously, during sterilization, portions having a molecular weight between 3 and 10 kDa are also removed.

**In** this manner, it is possible to obtain a biologically active fraction consisting exclusively of portions having a molecular weight lower than 3 kDa and between 10 and 30 kDa.

Preferably, in the sterilization stage, the portions having a molecular weight lower than 3 kDa and between 10 and 30 kDa are obtained separately.

**In** particular, in this regard, the clear solution may be subjected to sterilization and purification by means of a sequence of tangential ultrafiltration stages.

Thus, the sterilization stage may comprise a plurality of sub-stages, in particular a first, a second and a third ultrafiltration stage.

The first ultrafiltration stage is preferably carried out by means of a membrane having a 30 kDa cut-off in order to eliminate the high molecular weight portions.

Therefore, in this case, portions having a molecular weight greater than 30 kDa are removed while maintaining only portions having a molecular weight lower than 30 kDa.

The second ultrafiltration stage is preferably carried out by means of a membrane having a 3 kDa cut-off in order to separate portions below this threshold.

The third ultrafiltration stage then comprises filtration by means of a membrane having a 10 kDa cut-off.

The various filtration stages may be employed in different orders.

In particular, preferably, the first ultrafiltration stage is carried out first, but it could also be carried out subsequently.

In a preferred embodiment, the second and the third ultrafiltration stages are preferably carried out after the first filtration stage, but in any order. For example, the third ultrafiltration stage may be carried out before the second filtration stage.

If the second ultrafiltration stage is carried out before the third ultrafiltration stage, the second ultrafiltration stage separates portions having a molecular weight lower than 3 kDa from portions having a molecular weight between 3 and 30 kDa while retaining the portions having a molecular weight lower than 3 kDa, and the third tangential ultrafiltration stage is carried out on the clear solution including said portions having a molecular weight between 3 and 30 kDa by means of a membrane having a 10 kDa cut-off, in order to eliminate portions having a molecular weight lower than 10 kDa while retaining portions having a molecular weight between 10 and 30 kDa.

If, on the other hand, the second ultrafiltration stage is carried out after said third ultrafiltration stage, the third ultrafiltration stage separates portions having a molecular weight lower than 10 kDa from portions having a molecular weight between 10 and 30 kDa while retaining both the portions having a molecular weight lower than 10 kDa and the portions having a molecular weight between 10 and 30 kDa, and the third tangential ultrafiltration stage is carried out on the portions having a molecular weight lower than 10 kDa by means of a membrane having a 3 kDa cut-off, in order to eliminate portions having a molecular weight between 3 and 10 kDa while retaining portions having a molecular weight lower than 3 kDa.

In any case, and regardless of the order employed for ultrafiltration, substantially two distinct and separate fractions are obtained having respectively portions with a molecular weight lower than 3 kDa and portions with a molecular weight between 10 and 30 kDa.

The ultrafiltration stages are carried out in cascade and, therefore, starting from the clear solution or on clear solutions filtered in previous stages or alternatively on the portions filtered from the clear solution.

The process therefore comprises a combination stage. The latter is preferably subsequent to the sterilization stage.

In the combination stage, advantageously, the portions are mutually recombined. Naturally, the process may comprise an isolation stage.

The isolation stage is preferably a final isolation stage in which the fraction thus obtained is isolated from the clear solution. The isolation stage may obviously be incorporated into the set of sterilization and combination stages, for example if in the third ultrafiltration stage the portions having a molecular weight between 10 and 30 kDa are removed from the clear solution. In this case, the combination of the portions results in obtaining an isolated fraction.

Otherwise, if the portions having a molecular weight between 10 and 30 kDa remain in the clear solution, in the combination stage the recombination takes place in the clear solution and the isolation stage consists of a further filtration of the solution including all the portions.

In other words, depending on the order of the ultrafiltration stages, a fraction already isolated is directly obtained or a fraction to be isolated from the clear solution is obtained.

In any case, the biologically active fraction thus obtained may also be stabilized by the addition of a stabilizing agent selected from polyols or natural antioxidants.

The biologically active fraction obtained with the process according to the invention may be used for various purposes and for preparing particular compositions or formulations.

Therefore, the invention also includes the use of the fraction for the preparation of non-pharmacological or cosmetic topical compositions having antioxidant and moisturizing activity.

Furthermore, the invention also comprises the use of the fraction for the preparation of a topical medical device intended for the prevention or treatment of skin lesions, ulcers, burns or chronic wounds.

The invention also comprises a composition including the biologically active fraction. The composition may be in the form of a gel, cream, lotion or emulsion.

Furthermore, preferably, the fraction is present in a concentration between 5% and 10% by weight of the composition, in association with dermatologically compatible excipients.

An application example of the described invention is provided below.

The example provides:
1) Germination: 240 g of selected wheat are soaked for at least 4 hours in purified water in order to moisten the outer surface. After moistening, the seeds are evenly distributed on 500 g of a germination substrate (poplar sawdust), previously wetted with ≥ 2 L of purified water. The seeds are then allowed to germinate in the dark in a controlled germination chamber for 4-7 days, until the seedlings reach a height of 4-7 cm. The temperature of the germination chamber must be maintained between 10 and 20 °C with a minimum humidity of 60%.
2) Heat treatment: The content of the germination trays is placed in two containers and subjected to sterilization in an autoclave with a cycle corresponding to 60 minutes at 121 °C.
3) Maceration in water at a pH between 2 and 4 divided into several stages at room temperature (minimum 30 minutes) and between 7 and 9 °C (between 24 and 72 hours).
4) Heat treatment: The content of the germination trays is placed in two containers and subjected to sterilization in an autoclave with a cycle corresponding to 60 minutes at 121 °C.
5) Pressing: The liquid phase is extracted under a pressure of 298 bar, separating the liquid from the exhausted solid phase.
6) Ultrafiltration: In this stage, the fraction having a molecular weight cut-off (MWCO) < 30 kDa is collected using 30 kDa cassette filters.
7) From this ultrafiltrate, the following fractions are obtained: one with MW < 10 kDa and one with MW > 10 kDa.
8) Immediately after ultrafiltration, the fraction > 10 kDa is transferred into a container and mixed with a stabilizer.
9) The fraction < 10 kDa is loaded into a second ultrafiltration system equipped with a support containing four cassette filters having an MWCO of 3 kDa.
10) The two ultrafiltrates are subsequently combined.

With regard to antioxidant activity, the fraction according to the present invention may be used in the formulation of medicinal products for the treatment of wounds and ulcers, by virtue of its antioxidant properties.

In this regard, a test procedure used for the evaluation of antioxidant activity is described below, together with the corresponding results obtained.

The test procedure employed provides for the implementation of the Folin-Ciocalteu (F-C) test: the content of phenols/phenol-like and/or antioxidant compounds was determined by the F-C assay adapted to a 96-well microplate. A765 nm values were measured with a Synergy H4 microplate reader and compared with a gallic acid standard curve; the results were reported as mg of gallic acid equivalents (GAE) per liter and represent the mean of three independent experiments ± standard deviation (SD), each performed in triplicate.

ORAC assay The ORAC assay was performed using the Oxygen Radical Antioxidant Capacity (ORAC) activity assay. ORAC values were expressed as µmol Trolox Equivalents (TE) per gram of sample.

DPPH assay The α,α-diphenyl-β-picrylhydrazyl (DPPH) radical scavenging activity assay was determined as described. Gallic acid was used as the reference compound. The percentage of DPPH inhibition was calculated using the following formula: $Inhibition % = \left[\left(A0-At\right)/A0\times 100\right]$ wherein A0 and At are the absorbance values of the control (DPPH solution, without extract) and of the extract, respectively. The reported results are the mean of three independent experiments ± SD, each performed in triplicate.

The sheep erythrocyte hemolysis assay was performed using the following protocol: sheep erythrocytes were washed three times with 5 volumes of phosphate-buffered saline (PBS). Sheep erythrocytes at a final concentration of 5% were incubated with 4.4 mg/mL of the fraction obtained with the process according to the invention or with ascorbic acid, used as a reference, for 30 minutes at 37 °C. Subsequently, 200 mM AAPH was added to the mixtures and they were incubated at 37 °C; erythrocyte hemolysis was monitored every 20 minutes in the supernatants by spectrophotometric readings at A524 nm. The percentage of hemolysis was calculated using the formula: $hemolysis % = \left[\left(sample/positive control\right)\times 100\right] ;$ where the positive control consisted of a mixture of erythrocyte suspension and water (to obtain 100% hemolysis). The reported results, expressed as mean ± SD, are the mean of three independent experiments performed in triplicate. Statistical comparison between the reference compound and the Rigenase samples was carried out using one-way analysis of variance (ANOVA). A p value < 0.05 was considered statistically significant.

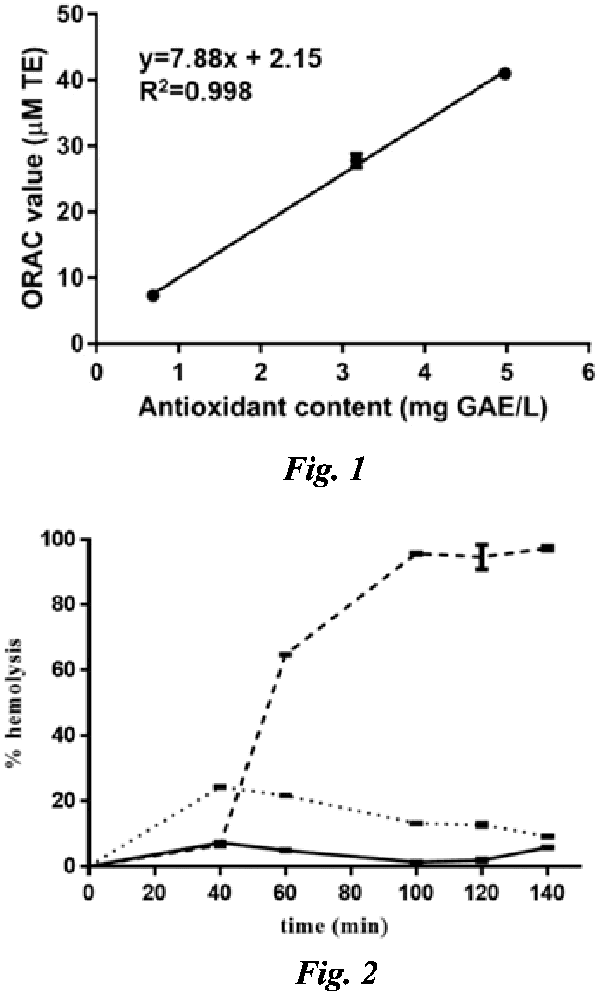

The results showed that the fraction according to the invention has an antioxidant compound content equal to 61.2 ± 3 mg GAE/L. To confirm the presence of antioxidant molecules, the fraction was also analyzed by the ORAC assay, as shown in Fig. 1. The correlation between the antioxidant compound content measured by F-C and the ORAC values of the biologically active fraction was determined using the regression analysis shown in Fig. 1. The determined correlation coefficient (R2) was 0.998, suggesting that the redox activity of the extract may be provided by phenolic/antioxidant compounds. To further investigate the antioxidant capacity, the DPPH assay was also performed on the fraction, as reported in the following table. The DPPH radical scavenging activity measured on the fraction was found to be comparable to that determined for several vegetable and fruit extracts.

| **Sample** | **µmol Trolox Equivalents (TE)/g** | **References** |
|---|---|---|
| Fraction | 74 ± 16 | [1] |
| Roasted Yak-kong (black beans) | 6.89 | [2] |
| Maqui fruits | 194-241 | [3] |
| Koji mold (*Aspergillus* sp.) | 38.3 | [4] |

The antioxidant activity of the fraction was also evaluated by means of the oxidative hemolysis inhibition assay. The obtained results reflect a biologically relevant radical scavenging activity and the microlocalization of antioxidants and are reported in the graph of Fig. 1.

With regard to moisturizing activity, similarly to antioxidant activity, a test procedure used for the evaluation of the antioxidant activity is described below, together with the corresponding results obtained.

In this case, protection from hyperosmotic stress is evaluated by assessing cell viability with the MTT assay on human keratinocyte monolayers in order to evaluate the moisturizing potential of a substance or mixture on human skin-derived keratinocytes. The cells are exposed to hyperosmotic stress in vitro by adding sodium chloride to the culture medium, which causes cell suffering and death due to dehydration. It is assessed whether cells treated with the sample at different concentrations show a higher viability rate compared to stressed and untreated cells. By adding known hygroscopic moisturizing ingredients to the cell culture medium, an increased capacity to retain and release water to the cells is obtained, thereby protecting them from stress-induced dehydration. The moisturizing effect of the sample is therefore measured indirectly by evaluating the protection of cell viability under stress, by means of the MTT assay. This colorimetric assay measures cell viability by monitoring the activity of an enzyme, mitochondrial succinate dehydrogenase, present only in living cells, which metabolizes the tetrazolium salt producing a colored reaction.

Treatment and exposure: cells were seeded in 96-well plates and allowed to grow for 24 hours at 37 °C and 5% CO2. On the second day, the cells were subjected to osmotic stress for 24 hours with NaCl 250 mOsm. On the third day, the culture medium was replaced with fresh medium supplemented with the tested product at concentrations of 5 and 2.5 mg/ml. The sample was dissolved directly in the culture medium. The test was carried out in three replicates for each dilution. As controls, stressed cells not treated with the sample (CDNT) and non-stressed untreated cells were used. At the end of the incubation period, the cells were subjected to the cytotoxicity test (MTT) to verify their viability.

MTT cell viability test: the culture medium is removed and the cells are incubated for 2 hours in a 1 mg/ml MTT solution at 37 °C. The solution is then removed and replaced with isopropanol, followed by a further 40 minutes of incubation at room temperature under medium-speed shaking. Absorbance at 570 nm is measured with a microplate reader (Tecan model Sunrise remote), with background compensation at 650 nm. Absorbance values are corrected by subtracting the blank reading, containing only the diluent. Cell viability is expressed as a percentage: 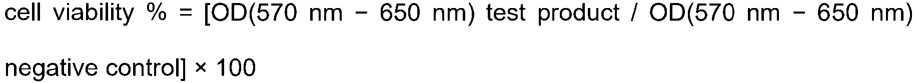 cell viability % = [OD(570 nm - 650 nm) test product / OD(570 nm - 650 nm) negative control] × 100
The cell death rate is then calculated as: cell death % = 100 - cell viability %
To evaluate protection of cell viability: % protection from dehydration = (CDNT cell death % - CDT cell death %) / CDNT cell death % × 100
Wherein CDNT = dehydrated cells not treated with the sample CDT = dehydrated cells treated with the sample

For the negative control: the mean OD value of the replicate must be > 0.6; the means of two blank columns must not differ by more than 15%. For the positive control (CP): the standard deviation must be ≤ 18%. For the sample: the standard deviation must be ≤ 18%.

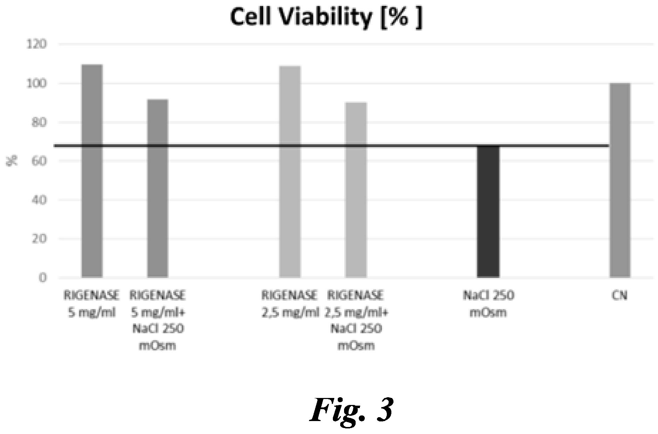

The test results are summarized in the table reported below and illustrated in the graph of Fig. 3 (wherein the Fraction is indicated as Rigenase).

| | [%] Cell viability (SD) | [%] Cell death | [%] Protection from dehydration |
|---|---|---|---|
| Fraction (5mg/mL) | 109.38 (1.29) | 0 | N/D |
| Fraction (5mg/mL)+ NaCl 250 MOsm | 91.74 (4.79) | 8.26 | 74.33 |
| Fraction (2.5mg/mL) | 108.44 (3.00) | 0 | N/D |
| Fraction (2.5mg/mL)+ NaCl 250 MOsm | 90.23 (4.22) | 9.77 | 69.62 |
| NaCl 250 mOsm CDNT* | 67.83 (5.05) | 32.17 | N/D |

| | | | |
|---|---|---|---|
| *Dehydrated cells not treated with the sample | | | |

Under the experimental conditions described above, the sample showed an in vitro dose-related protection against dehydration on human keratinocytes. The highest effect is observed at the concentration of 5 mg/ml (protection 74.33%).

The extraction and purification process of a biologically active fraction derived from common wheat according to the invention achieves important advantages.

Indeed, the extraction and purification process of a biologically active fraction derived from common wheat makes it possible to obtain an extract having high antioxidant, moisturizing and humectant capacities, as demonstrated by the tests carried out and reported above.

Furthermore, the extraction and purification process of a biologically active fraction derived from common wheat makes it possible to obtain a fraction particularly suitable for use in non-pharmaceutical compositions, for example in medical devices, and in cosmetic compositions, including antioxidant, moisturizing and humectant activities.

The invention is susceptible to variations within the inventive concept as defined by the claims.

Within this scope, all details may be replaced by technically equivalent elements, and the materials, shapes and dimensions may be any according to requirements.

## Claims

1. Process of extraction and purification of a biologically active fraction derived from common wheat having a molecular weight less than 30 kDa, comprising, in order, at least the following steps:
- germination of wheat seeds on water or a natural or artificial medium until sprouts are obtained;
- heat treatment of said sprouts obtaining treated sprouts;
- maceration in aqueous solution of said treated sprouts obtaining a solid residue;
- separation of said solid residue from said aqueous solution until a clear solution is obtained;
- sterilization of said clear solution by removing portions having molecular weights greater than 30 kDa;
and **characterized by the fact** that
- during said sterilization, portions with molecular weights between 3 and 10 kDa are also removed so as to obtain a biologically active fraction consisting exclusively of portions with molecular weights less than 3 kDa and between 10 and 30 kDa.

2. Process according to claim 1, wherein in said sterilization step said portions having molecular weights of less than 3 kDa and between 10 and 30 kDa are obtained separately and said process further comprises a combination step, subsequent to said sterilization step, wherein said portions are mutually recombined.

3. Process according to the previous claim, wherein said sterilization step includes:
- a first stage of tangential ultrafiltration using a membrane with a cut-off of 30 kDa, in order to eliminate said portions having a molecular weight greater than 30 kDa while maintaining said portions having a molecular weight less than 30 kDa,
- a second stage of tangential ultrafiltration using a 3 kDa cut-off membrane, and
- a third stage of tangential ultrafiltration using a 10 kDa cut-off membrane.

4. Process according to the preceding claim, wherein said second ultrafiltration step is carried out prior to said third ultrafiltration step, said second ultrafiltration step separating said portions with molecular weight less than 3 kDa from said portions with molecular weight between 3 and 30 kDa while retaining said portions with molecular weight less than 3 kDa, and said third tangential ultrafiltration step being carried out on said clear solution including said portions with molecular weight between 3 and 30 kDa by means of a membrane with cut-off of 10 kDa, in order to eliminate said portions having molecular weight less than 10 kDa while maintaining said portions with molecular weight between 10 and 30 kDa.

5. Process according to claim 3, wherein said second ultrafiltration step is carried out after said third ultrafiltration step, said third ultrafiltration step separating said portions with molecular weight less than 10 kDa from said portions with molecular weight between 10 and 30 kDa while retaining both said portions with molecular weight less than 10 kDa, and said portions with molecular weight between 10 and 30 kDa and said third tangential ultrafiltration step being carried out on said portions with molecular weight less than 10 kDa by means of a membrane with cut-off of 3 kDa, in order to eliminate said portions having molecular weight between 3 and 10 kDa while maintaining said portions with molecular weight less than 3 kDa.

6. Process according to any of the preceding claims, wherein said biologically active fraction is stabilized by the addition of a stabilizing agent selected from polyols or natural antioxidants.

7. Process according to any of the preceding claims, wherein said germination of wheat seeds is carried out in the absence of light on a lignocellulosic substrate selected from poplar sawdust or other inert natural substrates.

8. Process according to any of the preceding claims, wherein said germination stage is conducted at a temperature between 10 and 20 °C, with relative humidity above 60 percent, until said sprouts reach a height between 4 and 7 cm.

9. Process according to any of the preceding claims, wherein said heat treatment step is conducted in an autoclave at 121°C for 60 minutes.

10. Process according to any of the preceding claims, wherein said maceration step is conducted in aqueous solution with a pH between 2 and 4, at a temperature between 2 and 10 °C, for a period between 24 and 72 hours.

11. Process according to the preceding claim, wherein said step of separating said solid residue from said aqueous solution includes a coarse filtration step by pressing or mechanical filtration.

12. Use of a biologically active fraction obtained by a process according to any of the preceding claims for the preparation of a nonpharmacological or cosmetic topical composition with antioxidant and moisturizing activity.

13. Use of a biologically active fraction obtained by a process according to any of claims 1-8 for the preparation of a topical medical device intended for the prevention or treatment of skin lesions, ulcers, burns, or chronic wounds.

14. Topical composition for cutaneous use in the form of a gel, cream, lotion, or emulsion, comprising a biologically active fraction obtained by a process according to any of claims 1-9.

15. Composition according to the preceding claim, wherein said biologically active fraction is present in a concentration between 5% and 10% by weight of said composition, mixed with dermatologically compatible excipients.
